# EUROPÄISCHE PATENTANMELDUNG

(11) **EP 0 570 778 A1**
(43) Veröffentlichungstag der Anmeldung: **24.11.1993**
(21) Anmeldenummer: 93107416.5
(22) Anmeldetag: 07.05.1993
(51) Int. Cl.: C07D 311/92

(54) **Verfahren zur Herstellung von 4-(2-Aminophenylthio)-naphthalsäureanhydrid-Derivaten**

(30) Priorität: 21.05.1992 DE 4216796
(71) Anmelder: HOECHST AKTIENGESELLSCHAFT, D-65926 Frankfurt am Main (DE)
(72) Erfinder: Bauer, Wolfgang, Dr., W-6457 Maintal 3 (DE); Koumbouris, Wassilis, Dr., W-6000 Frankfurt am Main (DE)

(57) **Zusammenfassung**

Durch Umsetzung von 2-Aminothiophenolaten mit 4-Chloro- oder 4-Bromo-naphthalsäureanhydriden mit einer Partikelgröße von maximal 50 µm werden 4-(2-Aminophenylthio)-naphthalsäureanhydrid-Derivate in guten Ausbeuten und Reinheiten erhalten.

## Beschreibung

Die vorliegende Erfindung betrifft ein Verfahren zur Herstellung von 4-(2-Aminophenylthio)-naphthalsäureanhydrid-Derivaten der Formel I 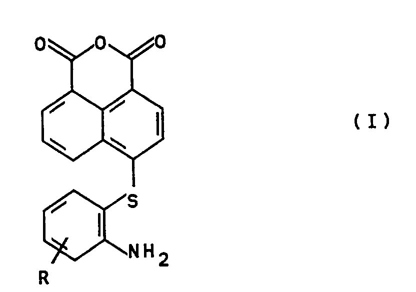
worin R = H, Methyl, Ethyl, Methoxy, Ethoxy oder Chlor bedeutet.

Die Verbindungen der Formel I sind wichtige Zwischenprodukte, die z.B. zur Herstellung von Fluoreszenzfarbstoffen benötigt werden, vgl. beispielsweise deutsche Patentschriften 2 447 024, 2 132 963, 2 134 517 sowie A.T. Peters et al, Tetrahedron 30, 2245 (1974). Um für die Herstellung derartiger Fluoreszenzfarbstoffe ohne weitere Reinigungsoperationen geeignet zu sein, müssen die Verbindungen der Formel I eine hohe Reinheit besitzen.

Zur Herstellung von in 4-Stellung durch Alkyl- oder Arylthioreste substituierten Naphthalsäureanhydrid-Derivaten sind bereits verschiedene nucleophile Austauschreaktionen von 4-Halogeno-naphthalsäureanhydrid-Derivaten mit Alkalimetall-thiolaten bekannt. Diesen bekannten Verfahren ist gemeinsam, daß die Ausbeuten und/oder Reinheiten unbefriedigend sind und daß sie darüberhinaus in organischen Lösungsmitteln durchgeführt werden müssen, wodurch erhebliche Kosten für die Wiedergewinnung der Lösungsmittel bzw. die Abwasserreinigung entstehen.

Nach P.H. Grayshan et al, J. Heterocycl. Chem. 11, 33-38 (1974), wird die Reaktion von 4-Chloronaphthalsäureanhydrid mit 2-Aminothiophenol und Kaliumcarbonat in Dimethylformamid durchgeführt. Dabei laufen jedoch unerwünschte Nebenreaktionen ab, so daß die entstandenen Nebenprodukte durch aufwendige, kostenintensive Reinigungsoperationen abgetrennt werden müssen. Die Ausbeute an 4-(2-Aminophenylthio)-naphthalsäureanhydrid beträgt nach diesem Verfahren lediglich ca. 60 % der Theorie. Neben der schlechten Ausbeute und dem Anfall unerwünschter Nebenprodukte ist dieses Verfahren wirtschaftlich nachteilig durch den Anfall eines Filtrats aus wäßrigem Dimethylformamid, wodurch hohe technische Aufwendungen durch Lösungsmittelregeneration und Abwassereinigungsmaßnahmen erforderlich sind. Die Verwendung von 2-Methoxyethanol liefert ähnliche Ergebnisse wie mit Dimethylformamid; in Ethanol verläuft die Reaktion unvollständig, verbunden mit der Bildung von großen Mengen an unerwünschtem 2,2-Diaminodiphenyldisulfid.

Auch bei der Umsetzung von 4-Chloronaphthalsäureanhydrid mit 1-Butanthiol und Kaliumcarbonat in Dimethylformamid wird lediglich eine Ausbeute von 72 % der Theorie erzielt (R.W. Middleton et al, J. Heterocycl. Chem. 22, 1567-1572 (1985)). Auch bei diesem Verfahren verursachen Nebenproduktbildung und Anfall von wäßrigem Dimethylformamid hohe Kosten im Hinblick auf Entsorgung von Nebenprodukten, Lösungsmittelregeneration und Gewässerschutz.

Die nach A. Peters et al, Dyes and Pigments 6, 267-275 (1985), in Ethanol als Lösungsmittel durchgeführte Reaktion von 4-Chloro-3-nitro-naphthalsäureanhydrid mit Thiophenol liefert 3-Nitro-4-phenylthio-naphthalsäureanhydrid mit einer Ausbeute von 57 % der Theorie. Auch bei diesem Verfahren sind aufwendige und kostenintensive Methoden erforderlich, um eine Belastung der Umwelt zu vermeiden. Die in der US-Patentschrift 3 850 965 beschriebenen Umsetzungen von 4-Halogeno-naphthalsäureanhydriden mit Alkalithiophenolaten werden z.B. in Dimethylformamid oder Dimethylsulfoxid und Benzol unter Abdestillation von Wasser durchgeführt. Auch bei diesem Verfahren treten die bereits geschilderten Nachteile auf. Hinzu kommt noch ein hohes Gesundheitsrisiko durch den Umgang mit Benzol.

Die Aufgabe der vorliegenden Erfindung war es daher, ein Verfahren zur Herstellung der 4-(2-Aminophenylthio)-naphthalsäureanhydrid-Derivate der Formel I durch Umsetzung von 4-Halogeno-naphthalsäureanhydriden der Formel II 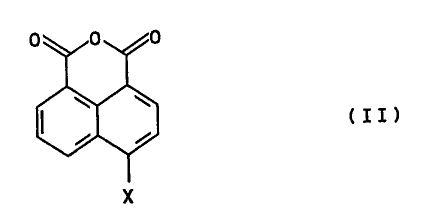
worin X Chlor oder Brom bedeutet, mit 2-Aminothiophenolaten der Formel III 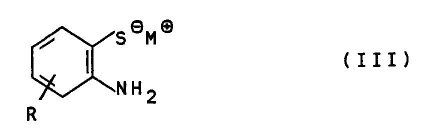
worin R die bereits genannte Bedeutung besitzt und M^{⊕} ein Alkalimetallkation, das Ammoniumion oder ein substituiertes Ammoniumion bedeutet, anzugeben, das die Nachteile der bisher bekannten Verfahren nicht besitzt, sondern es vielmehr gestattet, die Verbindungen der Formel I in wesentlich besseren Ausbeuten und Reinheiten ohne den Anfall unerwünschter Nebenprodukte herzustellen.

Die gestellte Aufgabe wird anspruchsgemäß gelöst. überraschenderweise wurde gefunden, daß bei der Umsetzung eines 4-Halogeno-naphthalsäureanhydrids der Formel II mit einem 2-Aminothiophenolat der Formel III dann die Verbindung der Formel I in wesentlich besserer Ausbeute und Reinheit erhalten wird, wenn das eingesetzte 4-Halogeno-naphthalsäureanhydrid der Formel II eine Teilchengröße von maximal 50 µm besitzt. Vorzugsweise besitzt das eingesetzte 4-Halogen-naphthalsäureanhydrid der Formel II eine Teilchengröße von maximal 40 µm und ganz besonders bevorzugt von maximal 25 µm.

Als 4-Halogeno-naphthalsäureanhydrid wird vorzugsweise 4-Chloro-naphthalsäureanhydrid eingesetzt, d.h. in der Formel II bedeutet X vorzugsweise Chlor.

In der Formel III bedeutet R vorzugsweise Wasserstoff. Für M^{⊕} ist ein Alkalimetallkation bevorzugt. Als Alkalimetallkatikon ist beispielsweise das Lithium-, Natrium-, Kalium-, Rubidium- oder Cäsiumkation zu nennen, von denen das Lithium-, Natrium- und Kaliumkation bevorzugt sind. M^{⊕} kann ferner das Ammoniumion NH₄^{⊕} oder ein substituiertes Ammoniumion sein. Substituierte Ammoniumionen sind z.B. solche, bei denen ein, zwei oder drei Wasserstoffatome des Ammoniumions NH₄^{⊕} durch gleiche oder verschiedene Alkylreste mit 1 bis 4 C-Atomen und/oder durch gleiche oder verschiedene Alkanolreste mit 2 bis 4 C-Atomen ersetzt sind. Derartige substituierte Ammoniumionen leiten sich z.B. von Mono-, Di- oder Trialkylaminen, wie z.B. Mono-, Di- und Triethylamin, Isopropylamin, Di-n-propylamin, Isobutylamin, Methylethylamin oder von Mono-, Di-und Trialkanolaminen, wie z.B. Ethanolamin, Diethanolamin, Triethanolamin, Dimethylethanolamin ab. Statt ein Alkalimetall- oder Ammonium-2-aminothiophenolat der Formel III einzusetzen, kann man auch das Alkalimetall- oder Ammonium-2-aminothiophenolat sich bei der Reaktion bilden lassen, d.h. man kann z.B. das entsprechende 2-Aminothiophenol (bei dem in Formel III M = H bedeutet) in Kombination mit einem Alkalimetallcarbonat, -bicarbonat oder -hydroxid oder einem Amin bzw. Alkanolamin einsetzen.

Das erfindungsgemäße Verfahren wird in einem geeigneten Lösungsmittel oder Lösungsmittelgemisch durchgeführt. Als geeignete Lösungsmittel kommen z.B. Alkohole, insbesondere Ethanol, Glykole, wie Ethylenglykol, Di- und Polyglykole, wie z.B. Diethylenglykol, Triethylenglykol, Glykolether, wie z.B. Methylglykol, Butylglykol, Methyldiglykol, Ethylenglykoldimethylether, Harnstoffderivate, wie z.B. Tetramethylharnstoff, Säureamide, wie z.B. Dimethylformamid, N-Methylpyrrolidon, oder Sulfoxide, wie z.B. Dimethylsulfoxid, in Betracht. Als ein Lösungsmittelgemisch kann z.B. Wasser im Gemisch mit einem oder mehreren mit Wasser teilweise oder vollständig mischbaren Lösungsmitteln verwendet werden. Beispiele für geeignete teilweise oder vollständig mit Wasser mischbare Lösungsmittel sind vorstehend aufgezählt. Da jedoch das erfindungsgemäße Verfahren überraschenderweise auch in Wasser hervorragende Ergebnisse liefert, ist die Durchführung des erfindungsgemäßen Verfahrens in Wasser ohne Zusatz organischer Lösungsmittel bevorzugt, um die durch das organische Lösungsmittel bedingten Nachteile, z.B. durch Lösungsmittelregeneration oder Abwasserbehandlung, zu vermeiden.

Die erfindungsgemäße Umsetzung kann z.B. bei einer Temperatur von 30 bis 150^{o}C, vorzugsweise von 50 bis 90^{o}C, durchgeführt werden. Bei der Umsetzung beträgt das Molverhältnis Verbindung II : Verbindung III vorzugsweise 1 : (1 bis 1,5).

Bei der Herstellung der 4-Halogeno-naphthalsäureanhydride der Formel II fallen diese Verbindungen mit Korngrößen bis zu hundert µm und mehr an. Aus derartigen Produkten können die für das erfindungsgemäße Verfahren benötigten Edukte der Formel II mit Teilchengrößen von maximal 50 µm in an sich bekannter Weise durch trockene oder nasse Teilchenzerkleinerung hergestellt werden. Derartige Zerkleinerungsverfahren können beispielsweise in geeigneten, an sich bekannten Mahl- oder Dispergiervorrichtungen, beispielsweise Sandmühlen, Perlmühlen, oder durch Anwendung von Ultraschall durchgeführt werden.

Es ist vorteilhaft, die Zerkleinerung in einem flüssigen, insbesondere wäßrigen Medium durchzuführen und dabei eine flüssige, insbesondere wäßrige Dispersion mit Teilchengrößen von maximal 50 µm, vorzugsweise maximal 40 µm und ganz besonders bevorzugt maximal 25 µm, herzustellen und das Edukt II in Form dieser flüssigen, insbesondere wäßrigen Dispersion bei dem erfindungsgemäßen Verfahren einzusetzen.

Bei der Durchführung des Zerkleinerungs- bzw. Dispergiervorgangs ist es vorteilhaft, den in einer Flüssigkeit, insbesondere im wäßrigen Medium, vorzugsweise in Wasser, zu dispergierenden Verbindungen der Formel II ein oder mehrere an sich bekannte Tenside zuzusetzen. Geeignete Tenside kommen beispielsweise aus der Reihe der anionischen und nichtionischen Tenside und werden beispielsweise in einer Menge von 0,001 % bis 1 Gew.%, bezogen auf die Verbindung der Formel II, eingesetzt. Eine Übersicht über geeignete nichtionogene oder anionische Tenside findet sich beispielsweise in "Ullmanns Enzyklopädie der Technischen Chemie", 4. Auflage, Band 10, Seite 449 ff., sowie Band 22, Seiten 455 ff., oder bei E.H. Daruwalla in K.Venkataraman "The Chemistry of Synthetic Dyes", Vol.VII, Seiten 86 bis 92 (1974).

Beispiele für besonders geeignete anionische Tenside sind: Ligninsulfonat, Alkansulfonate, Olefinsulfonate, Estersulfonate, Alkylarylsulfonate, Alkylsulfate, Ethersulfate, Fettalkoholsulfate sowie Phenolsulfonsäure-/ Formaldehyd- und Naphthalinsulfonsäure-/Formaldehyd-Kondensationsprodukte.

Besonders bevorzugte nichtionische Tenside stammen aus der Reihe der Alkylphenolpolyglykolether und deren Kondensationsprodukte mit Phenol und Formaldehyd sowie der Ethoxylierungsprodukte von Fettsäuren, Fettsäureamiden, Fettaminen und Fettalkoholen.

Die Verwendung einer vorzugsweise wäßrigen Dispersion des Edukts der Formel II mit einer Teilchengröße von maximal 50 µm, vorzugsweise maximal 40 µm und besonders bevorzugt maximal 25 µm, die vorzugsweise ein Tensid oder mehrere Tenside enthält, ist eine besonders bevorzugte Ausführungsform des erfindungsgemäßen Verfahrens.

Eine Dispersion des Edukts der Formel II kann entweder zu einer vorzugsweisen wäßrigen Lösung des 2-Aminothiophenolats der Formel III zugegeben, insbesondere zudosiert werden, oder der Dispersion des Edukts der Formel II wird eine insbesondere wäßrige Lösung von 2-Aminothiophenolat der Formel III zugegeben, insbesondere zudosiert.

Auch falls das 4-Halogeno-naphthalsäureanhydrid der Formel II nicht in Form einer ein Tensid enthaltenden Dispersion eingesetzt wird, kann es vorteilhaft sein, das erfindungsgemäße Verfahren in Gegenwart eines Tensids oder mehrerer Tenside durchzuführen. Auch hierfür kommen die bereits genannten Tenside in den bereits genannten Mengen in Betracht.

Das erfindungsgemäße Verfahren wird im wäßrigen Medium insbesondere bei einem pH-Wert von 4 bis 10, vorzugsweise bei einem pH-Wert von 5 bis 9, durchgeführt.

Die benötigten Ausgangsverbindungen der Formeln II und III sind bekannt bzw. können nach den für diese Verbindungsklassen bekannten Syntheseverfahren hergestellt werden.

Das erfindungsgemäße Verfahren, bei dem auf den Einsatz von organischen Lösungsmitteln und das Arbeiten im wasserfreien Medium verzichtet werden kann, liefert 4-(2-Aminophenylthio)-naphthalsäureanhydride der Formel I in überraschend hohen Ausbeuten von bis zu 95 % der Theorie und in einer so hohen Reinheit, daß sie ohne weitere Reinigungsoperationen direkt als Ausgangsprodukte bei der Synthese von Fluoreszenzfarbstoffen eingesetzt werden können.

Im Vergleich zu den bereits bekannten Verfahren bietet das erfindungsgemäße Verfahren deutliche Vorteile, insbesondere im Hinblick auf Ausbeute und Reinheit der Produkte, sowie im Hinblick auf Wirtschaftlichkeit und Umweltschutz.

In den nachstehenden Beispielen bedeuten Prozentangaben Gewichtsprozente.

### Beispiel 1

Eine Mischung von 232,6 g 4-Chloro-naphthalsäureanhydrid und 1 g Ligninsulfonat in 2,5 l Wasser wird in einer Labormühle vom Typ PUC-RD1 (Hersteller: Firma Probst & Class in 7550 Raststatt, Deutschland) 30 Minuten lang unter Umpumpen bei 25^{o}C aufgemahlen.

Eine Partikelgrößenanalyse (Meßmethode: Suspensionszelle, Ultraschall, Dauer 15 sec) ergibt, daß in der erhaltenen Suspension Teilchen von ca. 0,18 bis ca. 20,6 µm vorhanden sind. Das Maximum der Verteilungskurve liegt bei ca. 2,5 µm.

Der erhaltenen feinteiligen Dispersion, die einen pH-Wert von 5 aufweist, werden bei 25^{o}C 706 g einer 25%igen wäßrigen Natrium-2-aminothiophenolat-Lösung mit einem pH-Wert von 9 zugesetzt.

Anschließend heizt man das Reaktionsgemisch auf 80^{o}C und rührt 2 Stunden bei 80^{o}C nach.

Die gelbe Produktsuspension (pH-Wert: 6) wird filtriert, mit Wasser gewaschen und bei 100^{o}C getrocknet.

Ausbeute: 316 g gelbe Kristalle von 4-(2-Aminophenylthio)-naphthalsäureanhydrid mit einem durch Diazotierung bestimmten Gehalt von 96 %, entsprechend einer auf eingesetztes 4-Chloro-naphthalsäureanhydrid bezogene Ausbeute von 94,4 % der Theorie.

Schmelzpunkt: 196 bis 199^{o}C.

Das erhaltene Produkt ist ohne weitere Reinigungsoperationen hervorragend zur Synthese von Fluoreszenzfarbstoffen geeignet.

### Vergleichsbeispiel

232,6 g des auch in Beispiel 1 benutzten 4-Chloro-naphthalsäureanhydrids werden in ein Gemisch von 1 g Ligninsulfonat und 1535 ml Wasser eingetragen. Die erhaltene Suspension wird anschließend 30 Minuten bei 25^{o}C gerührt. Eine Partikelgrößenanalyse (Meßmethode: Suspensionszelle; Ultraschall, Dauer 15 sec) ergibt, daß 40 % der Teilchen eine Größe von 20 bis 100 µm aufweisen.

Anschließend wird die erhaltene Suspension nach den Angaben des Beispiels 1 mit 706 g einer 25%igen wäßrigen Natrium-2-aminothiophenolat-Lösung umgesetzt.

Ausbeute: 301,4 g gelbes Pulver mit einem Gehalt von 80 % 4-(2-Aminophenylthio)-naphthalsäureanhydrid entsprechend 75 % der Theorie, bezogen auf eingesetztes 4-Chloro-naphthalsäureanhydrid.
Schmelzpunkt: 168 bis 176^{o}C.

Das Produkt ist zur Herstellung von Fluoreszenzfarbstoffen ohne weitere Reinigungsoperationen nicht geeignet.

Der folgenden Tabelle sind weitere Beispiele für das erfindungsgemäße Verfahren zu entnehmen, die entsprechend Beispiel 1 durchgeführt werden, wobei in Spalte 2 Edukte der Formel II, in Spalte 3 Edukte der Formel III, in Spalte 4 das eingesetzte Tensid, in Spalte 5 die maximale Teilchengröße, in Spalte 6 das eingesetzte Lösungsmittel und in Spalte 7 die Ausbeute an den Produkten der Formel I, bezogen auf die Edukte der Formel II, angegeben ist.

Die in den Beispielen 2 bis 11 erhaltenen Produkte sind ohne weitere Reinigungsoperationen für die Herstellung von Fluoreszenzfarbstoffen hervorragend geeignet.

| Beispiel | Edukt der Formel II X = | Edukt der Formel III | | eingesetztes Tensid | Teilchengröße max.µm | Lösungsmittel | Ausbeute in % d.Th. |
|---|---|---|---|---|---|---|---|
| | | M^{⊕} | R | | | | |
| 2 | Br | Li^{⊕} | H | ®Tamol NNC ¹⁾ | 30 | Wasser | 92 |
| 3 | Cl | K^{⊕} | H | ®Hostapur SAS 93 ²⁾ | 40 | Wasser | 89 |
| 4 | Cl | Na^{⊕} | 3-CH₃ | Ligninsulfonat | 20 | Wasser | 90 |
| 5 | Cl | Na^{⊕} | 5-Cl | Ligninsulfonat | 20 | Wasser | 88 |
| 6 | Cl | Na^{⊕} | 2-OC₂H₅ | Ligninsulfonat | 20 | Wasser | 87 |
| 7 | Cl | Na^{⊕} | H | Emulgator W ³⁾ | 20 | Wasser | 90 |
| 8 | Cl | Na^{⊕} | H | Ligninsulfonat | 20 | Wasser/Ethanol (Gew.verh. 1:1) | 87 |
| 9 | Cl | NH₄^{⊕} | H | Ligninsulfonat | 25 | Wasser | 91 |
| 10 | Cl | HN^{⊕}(C₂H₅)₃ | H | Ligninsulfonat | 20 | Wasser | 90 |
| 11 | Cl | H₂N^{⊕}(C₂H₄OH)₂ | H | Ligninsulfonat | 20 | Wasser | 89 |

| | | | | | | | |
|---|---|---|---|---|---|---|---|
| 1.) Handelsprodukt der BASF AG, Ludwigshafen, Deutschland, auf der Basis Naphthalinsulfonat/Formaldehyd | | | | | | | |
| 2.) Handelsprodukt der HOECHST AG, Frankfurt/Main, Deutschland; sekundäres Alkansulfonat; | | | | | | | |
| 3.) Handelsprodukt der BAYER AG, Leverkusen, Deutschland; Arylpolyglykolether. | | | | | | | |

## Patentansprüche

1. Verfahren zur Herstellung von 4-(2-Aminophenylthio)-naphthalsäureanhydrid-Derivaten der Formel I 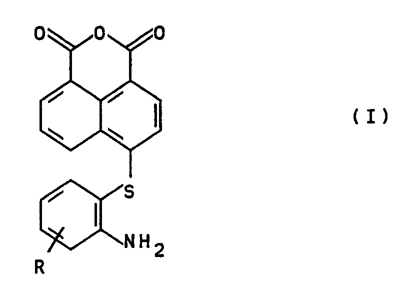 worin R = H, Methyl, Ethyl, Methoxy, Ethoxy oder Chlor bedeutet,
durch Umsetzung von 4-Halogeno-naphthalsäureanhydriden der Formel II 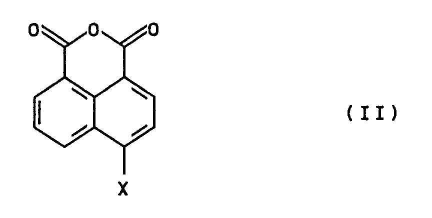 worin X Chlor oder Brom bedeutet, mit 2-Aminothiophenolaten der Formel III 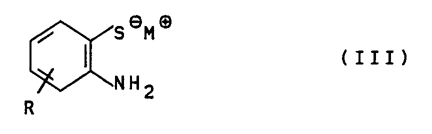 worin R die bereits genannte Bedeutung besitzt und M^{⊕} ein Alkalimetallkation, das Ammoniumion oder ein substituiertes Ammoniumion bedeutet, dadurch gekennzeichnet, daß das eingesetzte 4-Halogeno-naphthalsäureanhydrid der Formel II eine maximale Teilchengröße von 50 µm besitzt.

2. Verfahren nach Anspruch 1, dadurch gekennzeichnet, daß das eingesetzte 4-Halogeno-naphthalsäureanhydrid der Formel II eine maximale Teilchengröße von 40 µm, vorzugsweise von 25 µm, besitzt.

3. Verfahren nach Anspruch 1 und/oder 2, dadurch gekennzeichnet, daß eine Verbindung der Formel II eingesetzt wird, in der X Chlor bedeutet und/oder daß eine Verbindung der Formel III eingesetzt wird, in der R Wasserstoff und/oder M^{⊕} ein Alkalimetallkation, insbesondere das Lithium-, Natrium- oder Kaliumkation bedeuten.

4. Verfahren nach einem oder mehreren der Ansprüche 1 bis 3, dadurch gekennzeichnet, daß die Umsetzung bei einer Temperatur von 30 bis 150^{o}C, vorzugsweise 50 bis 90^{o}C, durchgeführt wird und/oder das Molverhältnis zwischen der Verbindung II und der Verbindung III = 1 : (1 bis 1,5) beträgt.

5. Verfahren nach einem oder mehreren der Ansprüche 1 bis 4, dadurch gekennzeichnet, daß das 4-Halogeno-naphthalsäureanhydrid der Formel II in Form einer flüssigen, vorzugsweise wäßrigen Dispersion eingesetzt wird, die vorzugsweise ein Tensid oder mehrere Tenside enthält.

6. Verfahren nach einem oder mehreren der Ansprüche 1 bis 5, dadurch gekennzeichnet, daß die Umsetzung in Gegenwart eines Tensids oder mehrerer Tenside durchgeführt wird.

7. Verfahren nach einem oder mehreren der Ansprüche 1 bis 6, dadurch gekennzeichnet, daß die Umsetzung in einem Gemisch aus Wasser und einem oder mehreren mit Wasser teilweise oder vollständig mischbaren Lösungsmitteln durchgeführt wird.

8. Verfahren nach einem oder mehreren der Ansprüche 1 bis 7, dadurch gekennzeichnet, daß die Umsetzung in Wasser durchgeführt wird.

9. Verfahren nach einem oder mehreren der Ansprüche 1 bis 8, dadurch gekennzeichnet, daß die Umsetzung bei einem pH-Wert von 4 bis 10, vorzugsweise 5 bis 9, durchgeführt wird.

10. Verwendung eines nach einem Verfahren einer oder mehrerer der Ansprüche 1 bis 9 hergestellten 4-(2-Aminophenylthio)-naphthalsäureanhydrids der Formel I zur Herstellung von Fluoreszenzfarbstoffen.
